(19) Europäisches Patentamt
European Patent Office
Office européen des brevets

(11) EP 1 338 283 A1

(12) **DEMANDE DE BREVET EUROPEEN**

(43) Date de publication:
**27.08.2003 Bulletin 2003/35**

(51) Int Cl.[7]: **A61K 31/7048**, A61K 35/78, A61K 7/06, A61P 17/14

(21) Numéro de dépôt: **03290452.6**

(22) Date de dépôt: **26.02.2003**

(84) Etats contractants désignés:
**AT BE BG CH CY CZ DE DK EE ES FI FR GB GR HU IE IT LI LU MC NL PT SE SI SK TR**
Etats d'extension désignés:
**AL LT LV MK RO**

(30) Priorité: **26.02.2002 FR 2002400**

(71) Demandeur: **Pierre Fabre Dermo-Cosmetique**
**92100 Boulogne (FR)**

(72) Inventeurs:
- **Belle René**
**81600 Rivieres (FR)**
- **Dunouau Christophe**
**31130 Balma (FR)**
- **Fabre Bernard**
**31450 Belberaud (FR)**
- **Charveron Marie**
**31000 Toulouse (FR)**
- **Lachgar Souad**
**31520 Ramonville Saint Agne (FR)**

(74) Mandataire: **Le Forestier, Eric et al**
**Cabinet Régimbeau**
**20, rue de Chazelles**
**75847 Paris cedex 17 (FR)**

(54) **Utilisation de néoruscine pour traiter l'alopecie**

(57) Utilisation de la néoruscine pour la fabrication d'une composition capillaire destinée au traitement de l'alopécie.

Méthode de traitement cosmétique pour lutter contre la chute des cheveux consistant à administrer par voie topique une composition capillaire contenant de la néoruscine.

EP 1 338 283 A1

## Description

La présente invention a pour objet l'utilisation de la néoruscine pour la préparation d'une composition capillaire destinée au traitement de l'alopécie.

L'alopécie se définie comme étant la chute temporaire ou définitive, totale ou partielle, des cheveux ou des poils, qui est due à l'âge, à des facteurs génétiques ou qui fait suite à une affection locale ou générale.

L'alopécie peut avoir différentes causes d'origines internes ou externes.

Par origines internes, on entend toutes perturbations touchant les processus biologiques impliqués dans la croissance et le maintien du cheveu. On citera par exemple le métabolisme hormonal, le dérèglement sébacé, l'amplification enzymatique des 5-$\alpha$-réductases, l'inflammation, les modifications structurales du collagène, l'intervention des facteurs de croissance, des facteurs nerveux ou des facteurs vasculaires et la composante génétique.

Par origines externes, on entend l'incidence de la flore locale du cuir chevelu, l'action d'agents chimiques (colorations, permanentes, chimiothérapie), la pollution ou l'hygiène de vie.

Tous ces désordres, d'origines internes ou externes, ciblant le cuir chevelu, ont pour conséquence directe la perturbation et la modification du cycle pilaire conduisant à différentes formes d'alopécies.

Le terme d'alopécie résume l'ensemble des pathologies atteignant le follicule pileux, son environnement, ainsi que le cycle de croissance de la tige pilaire. On citera les 2 types d'alopécies les plus répandus, l'Alopécie Aérata et l'Alopécie *Androgénogénétique ou Androgénique.*

Depuis longtemps, les scientifiques recherchent à travers différentes voies biologiques, les solutions visant à stimuler la croissance du cheveu ou à prolonger son attachement au cuir chevelu. Cette préoccupation est d'autant plus marquée dans le domaine de l'alopécie androgénique et plus particulièrement fréquente chez les sujets masculins, chez lesquels on constate une perturbation du cycle de croissance pilaire, entraînant la chute du cheveu.

Le follicule pileux constitue l'unité biologique fondamentale, directement impliquée dans le développement et le maintien du système pilaire. Il rassemble différentes entités structurales telles que la papille dermique, la glande sébacée, les gaines épithéliales, l'innervation ou la vascularisation impliquées séparément ou conjointement dans le cycle de développement des cheveux. Le lien existant entre la papille dermique et le bulbe reste déterminant dans la croissance du cheveu (cf. Olivet RF. Histological studies of whisker regeneration in the hooded rat. J Embryol Exp Morphol 1966; 311. 560-2 et Jahoda CAB. Home KA. Olivet RF. induction of hair growth by implantation of cultured dermal papilla cells. Nature 1966 ; 311. 560-2.

Le cycle de croissance pilaire est divisé en trois phases principales :

- une PHASE ANAGENE active phase de croissance pendant laquelle le follicule s'enfonce dans les tissus dermiques. Les cellules du bulbe entament une mitose rapide suivie d'une différenciation. La phase anagène est reconnue aujourd'hui comme l'étape primordiale de la croissance du cheveu, son accélération expliquant le raccourcissement de la vie du cheveu et sa rapidité de chute.
- une PHASE CATAGENE au cours de laquelle les divisions cellulaires régressent puis s'arrêtent. A ce stade, le follicule pileux amorce une remontée extra-dermique et une rétraction, c'est la phase la plus courte estimée à environ 3 semaines.
- une PHASE DE REPOS OU PHASE TELOGENE pendant laquelle le cheveu est progressivement délogé par un nouveau cheveu anagène issu du même follicule. Le cheveu natif va provoquer la chute du cheveu mature.

Il existe une perte de cheveux normale et physiologique de l'ordre de à 100 par jour pour une chevelure saine. Au-delà, la chute est dite pathologique qu'elle soit occasionnelle ou installée.

A ce jour, on a proposé différents produits pour lutter contre les deux types d'alopécies. La plupart associent plusieurs principes actifs susceptibles d'apporter une action bénéfique sur les paramètres biologiques mis en cause dans la chute des cheveux.

Parmi les principes actifs les plus couramment rencontrés, nous pouvons citer à titre d'exemples

- des vitamines telles que les vitamines A, E, B5, B6, C, H, PP.
- des oligo-éléments tels que le zinc, le cuivre, le magnésium, le silicium,
- des dérivés protéiques tels que les peptides, les acides aminés soufrés (type méthionine, cystine, cystéïne ou dérivés),
- des huiles essentielles ou des extraits d'origine végétale dont la liste n'est pas limitative,
- des agents antifongiques tels que la piroctonolamine, les dérivés undécyléniques, la cyclopiroxolamine,
- des molécules de synthèse chimique réputées pour leur action spécifique au niveau des récepteurs androgéniques ou sur la synthèse ou l'expression des 5-$\alpha$-réductases.

Parmi les dernières découvertes effectuées ces dernières années, le MINOXIDIL® (ou 2,4 diamino-6-pipéridinoperimidine 3-oxide) fait aujourd'hui référence dans le traitement de l'alopécie androgénique. En dépit des nombreuses théories évoquées sur son mécanisme d'action, ce dernier n'est pas clairement élucidé. De plus, son efficacité reste limitée, même s'il est constaté une stabilisation de la chute dans de nombreux cas cliniques en raison de la reprise du processus alopéciant dès l'arrêt du traitement. Son usage journalier contraignant est vraisemblablement à l'origine d'effets secondaires indésirables relevés chez des patients l'utilisant à long terme tels que réactions cutanées (ovalisées ou effets systémiques.

On a par ailleurs proposé dans les demandes de brevet WO 9501197 et WO 9201437 une nouvelle molécule inspirée de la précédente la 2,4 diamino-pyrimidine 3-oxide ou AMINEXIL®.

L'AMINEXIL® agit contre la fibrose périfolliculaire en inhibant l'expression de la lysylhydroxylase, elle-même responsable de la transformation accélérée de l'hydroxylysine conduisant à la formation précoce de collagène mature. Cette surproduction de collagène et simultanément sa réticulation entraînent une rigidification de la gaine conjonctive et une fibrose périfolliculaire s'opposant aux processus de formation des néo-cheveux.

Tous ces différents traitements proposés restent insuffisants parce que ne s'attaquant bien souvent qu'à l'une des causes directement impliquées dans le phénomène de alopécie.

La néoruscine possède la formule chimique suivante :

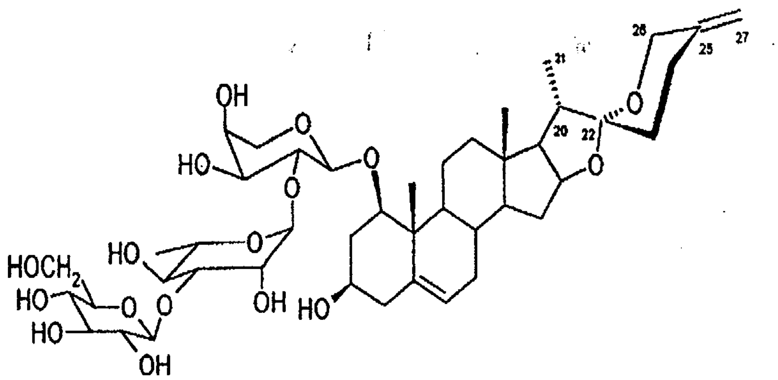

NEORUSCINE

La demanderesse a maintenant trouvé que la néoruscine stimule la production du facteur de croissance vasculaire endothélial (VEGF). La présente invention a donc pour objet l'utilisation de la néoruscine pour la préparation d'une composition capillaire destinée au traitement de l'alopécie.

En effet, la chute du cheveux semble être associée à une disparition du réseau capillaire sanguin.

Ainsi, la croissance de la tige pilaire dépend de la prolifération et des facteurs cytosolubles des cellules du bulbe folliculaire. L'expression de certains facteurs de croissance varie au cours du cycle pilaire, d'autre part, on note aussi un changement de la vascularisation en fonction du cycle pilaire. Si la croissance du poil dépend de plusieurs facteurs, les facteurs susceptibles d'induire une angiogénèse sont des candidats de choix dans l'induction et le maintien de la vascularisation au niveau de la papille dermique car on note une disparition du réseau capillaire de la papille durant le stade télogène.

Des tests biologiques ont permit de mettre en évidence l'utilité de la néoruscine dans le traitement de l'alopécie.

Les résultats de ces essais montrent que la néoruscine peut être utilisée pour la fabrication de compositions capillaires destinées au traitement de l'alopécie sous toutes ces formes.

A cet effet, la néoruscine peut être présentée sous toutes formes de compositions dermatologiques, cosmétiques ou dermocosmétiques adaptées à l'administration topique, en association avec des excipients appropriés.

Suivant le degré de l'affection, l'administration journalière pourra varier 1 à 10 mg.

Pour les affections légères, une application d'une composition capillaire, à titre de traitement cosmétique pourra être suffisante et l'administration journalière n'excèdera pas de 1 à 3 mg. Un tel traitement cosmétique pour lutter contre la chute des cheveux fait également partie de l'invention.

En revanche, pour les affections entraînant une chute massive des cheveux, le traitement pourra être considéré comme un traitement véritablement dermatologique et l'administration journalière pourra varier de 5 à 10 mg.

Les sérums, lotions, gels, shampoings, mousses, émulsion huile-dans-l'eau, émulsion eau-dans-l'huile éventuellement bi ou triphasiques, sont notamment adaptés pour la mise en oeuvre de l'invention.

La composition capillaire utile pour la mise en oeuvre de l'invention peut en outre renfermer au moins un adjuvant utilisé habituellement dans les compositions capillaires.

Parmi ces adjuvants, on peut citer les agents conservateurs, les agents séquestrants, les agents antioxydants, les parfums, les tensioactifs non ioniques, les filtres solaires, les stabilisateurs de mousse, les épaississants.

Parmi les agents tensioactifs anioniques qui peuvent être utilisés seuls ou en mélanges, on peut citer en particulier les sels alcalins, les sels d'ammonium, les sels d'amine ou les sels d'amino-alcool des composés bien connus tels que les alcoyles sulfates, les alcoyles éther sulfates, les alcoyles amide sulfates, les alcoyles éther sulfosuccinates.

Parmi les agents tensioactifs non ioniques qui peuvent être utilisés seuls ou en mélange on peut citer en particulier : les alcools, les alcoylphénols et acides gras polyéthoxylés, polypropoxylés ou polyglycérolés à chaîne grasse comportant 8 à 18 atomes de carbone ou les tensioactifs du type Cremophor RH 40 ®.

Comme épaississant, on peut citer les polysiloxanes. Parmi les agents liants, on peut citer les huiles de silicone volatiles ou non.

On obtient notamment la néoruscine par extraction du *Ruscus aculeatus,* appelé communément Petit Houx.

On peut notamment décrire le procédé suivant qui permet d'obtenir des extraits de *Ruscus* enrichis en néoruscine.

On extrait les rhizomes de *Ruscus* secs et broyés dans un alcool de C1 à C4 seul ou en mélange toute proportion avec de l'eau. Le rapport plante/solvant peut varier de 1/3 à 1/20. On réalise l'extraction sous agitation à une température variant de la température ambiante à la température de reflux pendant une durée variant de 1 h à 24 h. Une fois l'extraction réalisée, on récupère les solutions par filtration ou par centrifugation. Une deuxième extraction peut être réalisée sur les marcs en respectant les mêmes conditions que la première. Les 2 solutions sont alors jointes.

Les solutions extraites peuvent être concentrées ; on rajoute à ce concentrât un alcool de C2 à C4 plus apolaire que le solvant d'extraction. L'alcool utilisé est préférentiellement un alcool en C3 à C4 tel que le butanol ou l'isopropanol. On filtre alors le précipité apparu pour évaporer à sec le filtrat obtenu, laissé sous forme de poudre ou repris par un solvant cosmétiquement compatible comme l'alcool, le propylène glycol, le butylène glycol.

Les extraits obtenus sont dosés pour leur teneur en néoruscine par CLHP. Les teneurs en néoruscine se situent entre 1 et 10 pour 100 g de matière sèche de l'extrait.

Ainsi, l'invention a également pour objet l'utilisation de la néoruscine pour la préparation d'une composition destinée au traitement de l'alopécie, caractérisée en ce que la néoruscine est préparée par extraction à partir du *Ruscus aculeatus*.

Un exemple est fourni plus loin pour illustrer des compositions capillaires contenant la néoruscine notamment extraite du *Ruscus aculeatus.*

La figure 1 se rapporte aux résultats de l'expérience exposée à l'exemple 4 ci-après.

**EXEMPLE 1 - Dosage du VEGF**

Nous nous sommes intéressés en particulier au facteur de croissance angiogénique, le VEGF, qui est le seul facteur de croissance angiogénique qui est mitogène et qui est sécrété par les cellules endothéliales vasculaires, et représentatif de l'angiogénèse *in vitro.*

Les cellules de la papille dermique du follicule pileux présentent des récepteurs à ce facteur et le produisent in vitro (Lachgar et al., J. Invest Dermatol, 1996, 107 : 1723). La protéine et les ARN messagers du VEGF, sont également exprimés au niveau des cellules de la papille dermique durant le stade anagène (Lachgar et al., In "Hair for the next millenium". Eds D.J.J. Van Neste, V.A. Randall, H. Baden, H. Ogawa, R. Oliver. Elsevier, Amsterdam. 1996, pp. 407-412).

La suspension de cellules de la papille dermique ($10^6$ cellules/2 ml) dans le DMEM 10 % SVF est distribuée dans les plaques 6 puits ($10^6$ cellules/puits) et incubée pendant 18 heures à 37°C dans une atmosphère à 5 % $CO_2$. Les cellules de la papille folliculaire sont alors rincées avec du PBS pour éliminer les cellules non adhérentes puis exposées pendant 24 heures aux produits à tester inclus dans du DMEM sans SVF (qui pourrait interférer dans le dosage). Pour chaque produit testé, 3 puits sont réalisés.

L'extrait de *Ruscus* et la néoruscine purifiée sont testés en culture.

Après 24 heures de traitement, les surnageants sont récupérés, centrifugés puis conservés à -80°C avant leur utilisation ultérieure pour le dosage du VEGF.

La production du VEGF dans les surnageants est mesurée par Kit Elisa (R&D Biosystems). Les résultats sont exprimés en picogrammes de VEGF par $10^6$ cellules et en pourcentages d'activité.
Dans ce test la néoruscine à 50 µg/ml stimule la production du VEGF de 329 %.

## EXEMPLE 2 - Étude de l'expression du gène VEGF

La suspension de cellules de la papille folliculaire ($10^6$ cellules par boîte de 25 $cm^2$) dans le milieu DMEM est distribuée dans les boîtes de culture puis incubés pendant 12 heures à 37°C dans une atmosphère à 5 % de $CO_2$. Les CPD sont ensuite rincées avec du PBS pour éliminer les cellules non adhérentes.

Pour chaque lot, les cellules sont pré-incubées pendant 3 heures avec les produits à tester préparés dans du DMEM. Les cellules sont ensuite rincées deux fois au PBS.

### Extraction des ARN totaux

Après rinçage au PBS, les boîtes de culture sont déposées sur de la glace. Les cellules sont traitées avec la solution d'extraction d'ARN, RNAβTM (1 ml/boîte).

Les lysats cellulaires sont ensuite récupérés, centrifugés après avoir ajouté du chloroforme.
Trois phases sont alors observables

- inférieure contenant les débris cellulaires - interface contenant l'ADN
- interface contenant l'ADN
- supérieure avec l'ARN

Les surnageants sont prélevés, les ARN sont précipités avec de l'isopropanol. Après centrifugation, les ARN sont lavés à l'éthanol 75 %, centrifugés, séchés sous vide puis dissous dans de l'eau dépourvue de RNases.

La quantité d'ARN ainsi que la pureté sont déterminées par spectrophotométrie (Chomczynski P., Sacchi N. Anal. Biochem. 1987 ; 162: 4656-459).

### Amplification des ARNm du VEGF par RT-PCR

L'ARN total (1 µg) est rétro-transcrit en ADN complémentaire une heure à 48°C avec le kit Access RT-PCR system (Promega).

L'amplification se fait sur 40 cycles à l'aide d'un thermocycleur (1,5 min à 94°C, 2 min à 72°C, 1 min à 94°C), avec des oligonucléotides complémentaires des régions 3' et 5' terminales de la séquence codante pour le VEGF ou pour le cytochrome P450 aromatase.

Les produits d'amplification sont analysés par électrophorèse (50V pendant 45 min) sur un gel à 3 % d'agarose. Les bandes sont détectées après coloration au bromure d'éthidium à 1 µg/ml.

Une caméra UV permet de saisir l'image du gel et la quantification du volume et de l'intensité des bandes correspondant à l'ADNc du VEGF se fait à l'aide d'un logiciel d'analyse d'image.

Le traitement des cellules de la papille dermique avec la néoruscine à une concentration de 50 µg/ml entraîne une stimulation de la transcription du gène du VEGF de 24,5 %.

## EXEMPLE 3 - Etude de l'expression du gène du cytochrome p450 - aromatase.

Nous avons évalué, dans le cadre de cet exemple, l'activité de la néoruscine sur la transcription du gène VEGF par les cellules de la papille dermique humaine en culture.

Les cellules de la papille dermique sont ensemencées à $10^6$ cellules par boîte dans du DMEM à 10 % de SVF et 1 % de Pénicilline/Streptomycine pendant 24 heures à 37°C dans une atmosphère à 5 % $CO_2$. Les cellules de la papille folliculaire sont alors rincées avec du PBS pour éliminer les cellules non-adhérentes puis exposées aux produits à tester dans du DMEM sans SVF (qui pourrait interférer dans le dosage) durant 3 heures.

Les principes actifs ont été testés en culture à des concentrations non cytotoxiques. Une boîte de culture est réalisée pour chaque lot.

### Extraction des ARN totaux

Après rinçage au PBS, les boîtes de culture sont déposées sur de la glace. Les cellules sont traitées avec la solution d'extraction d'ARN, RNA β*TM* (1 ml/boîte). Les lysats cellulaires sont ensuite récupérés, centrifugés après avoir ajouté du chloroforme.
Trois phases sont alors observables

- inférieure contenant les débris cellulaires
- interface contenant l'ADN

- supérieure avec l'ARN

Les surnageants sont prélevés, les ARN sont précipités avec de l'isopropanol. Après centrifugation, les ARN sont lavés à l'éthanol 75 %, centrifugés, séchés sous vide puis dissous dans de l'eau dépourvue de RNases. La quantité d'ARN ainsi que la pureté sont déterminées par spectrophotométrie.

**Amplification des ARNm du P-450-aromatase par RT-PCR**

L'ARN total (1 µg) est rétro-transcrit en ADN complémentaire une heure à 48°C avec le kit Access RT-PCR system (Promega).
Pour le cytochrome P450 aromatase, l'amplification se fait sur 35 cycles.
Les produits d'amplification sont analysés sur gel à 3 % d'agarose. Les bandes sont détectées après coloration au bromure d'éthidium à 1 µg/ml.
Les produits d'amplification sont analysés par électrophorèse (50V pendant 45 min) sur un gel à 3 % d'agarose.
Une caméra UV permet de saisir l'image du gel et la quantification du volume et de l'intensité des bandes correspondant à l'ADNc et du cytochrome P450 aromatase se fait à l'aide d'un logiciel d'analyse d'image.
Dans ce test la néoruscine à une concentration de 5 µg/ml stimule la transcription du gène cytochrome P450 aromatase de 81 %.

**EXEMPLE 4 - Effet de la néoruscine sur la croissance du follicule pileux humain *ex vivo* après 5 jours de traitement.**

**MATERIEL ET METHODES**

**Culture:**

Les follicules pileux sont issus de scalp humain provenant du service de chirurgie plastique des hôpitaux ou des cliniques. La peau est rasée puis lavée dans 10 bains successifs de 10 min de PBS supplémentés d'antibiotiques (pénicilline 100 unités/ml et streptomycine 100 µg/ml). Les follicules en phase anagène reconnaissables car très vascularisés sont ensuite isolés de la peau sous la loupe binoculaire et débarrassés de la graisse. Ils sont ensuite incubés à 37°C dans des boîtes de culture dans du milieu de culture DMEM contenant le produit à tester. Le milieu de culture des follicules ne contient pas de sérum. Ce milieu est composé de 10µg/ml d'insuline, de 10 µg/ml de transferrine, de 10 µg/ml d'hydrocortisone, de 1% d'un mélange de pénicilline (100 uités /ml)-streptomycine (100µg/ml), de 2,5 µg/ml de fungizone et de la L-glutamine à 1mM.

**Traitement des follicules pileux**

Les follicules sont mis en présence de différentes concentrations de néoruscine (50, 100 et 300 µg/ml) (10 follicules par traitement). Les témoins sont cultivés dans du milieu sans produit.

**Mesures de la croissance:**

La croissance de la tige des follicules pileux est mesurée à l'aide de papier millimétré sous loupe binoculaire au jour 0 et tous les jours pendant 5 jours. Une caméra numérique permet de saisir les images de follicules pileux et de calculer avec précision leur taux de croissance.

Le pourcentage de croissance = (mesure Tx - mesure T0)/ mesure T0).

L'activité du traitement = (mesure traité Tx - mesure Témoin Tx)/mesure Témoin Tx).

(Tx= temps x après traitement, T0= temps 0 début de traitement)
La croissance des follicules pileux humains traités pendant 5 jours avec la néoruscine à 100 et 300 µg/ml est significativement supérieure à celle des témoins (+ 77,08% et 118,09% d'activités respectives).
Les résultats de cette expérience sont rassemblés à la figure 1.

**EXEMPLE 5 - Préparation d'un extrait enrichi en néoruscine et formulation de compositions capillaires.**

**Extraction 1**

Une tonne de rhizomes de *Ruscus* secs et broyés est extraite par 10 tonnes d'alcool 95 % pendant 1 h sous reflux. La solution est récupérée par filtration, le marc est de nouveau extrait dans les mêmes conditions, la solution est récupérée par filtration.

Les 2 solutions jointes sont concentrées jusqu'à l'obtention de 4 tonnes de concentrat. On rajoute alors 4 tonnes de butanol-1. On filtre le précipité. La solution obtenue est concentrée à sec puis reprise dans une tonne d'alcool 95 %. L'extrait obtenu limpide est dosé à 2,5 % en néoruscine par rapport à la matière sèche.

**Extraction 2**

100 kg de rhizomes de *Ruscus* secs et broyés sont extraits à température ambiante et sous agitation durant 24 h avec 1 tonne de méthanol. La solution récupérée après filtration est concentrée jusqu'à 100 kg. On rajoute 100 kg d'isopropanol. Le précipité obtenu est éliminé par filtration. La solution est évaporée jusqu'à l'obtention d'une poudre titrant à 5 % en néoruscine.

| **Sérum antichute** | |
|---|---|
| Extrait de Ruscus à 2,5% de néoruscine | 0,5 à 2% (extraction 1) |
| Provitamine B5 | 0,3% |
| Gluconate de zinc | 0,10% |
| Pyridoxine chlorhydrate | 0,10% |
| Phytopeptides | 1% |
| Extrait de Quniquina | 0,75% |
| Alcool 95 | 16 % vol. |
| Cremophor RH 40 (BASF) | 0,60% |
| Ethoxydiglycol | 2% |
| Eau purifiée QSP | 100 ml |

| **Sérum antichute bi-phasique** | |
|---|---|
| Phase lipophile | |
| Extrait de Serenoa repens | 0,5 g |
| Huile essentielle de Pin | 5 g |
| Cyclomethicone D5 | 35 g |
| Phase polaire | |
| Hexylène glycol | 10 g |
| Dimethicone copolyol | QS |
| Extrait de Ruscus à 2,5% de néoruscine | 6 g (3 à 10 g) (extraction 1) |
| Alcool 95° | 44 ml |
| Parfum | QS |

| **Lotion capillaire** | |
|---|---|
| Extrait de Ruscus à 2,5 % de néoruscine | 0,5 à 2% (extraction 1) |
| Piroctonolamine | 0,05% |
| Extrait fluide de Capucine | 1% |
| Dimethicone copolyol | 0,6% |
| Terpènes d'Orange | 0,03% |
| Huile essentielle de Romarin | 0,05% |

(suite)

| Lotion capillaire | |
|---|---|
| Chlorure de cetrimonium | 0,5% |
| Alcool | 50 % VOL. |
| Eau purifiée QS | 100 ml |

| Shampooing antichute | |
|---|---|
| Extrait liposterolique de Sabal | 0,3 g |
| Extrait de Ruscus à 2,5 % de néoruscine | 3 g (extraction 1) |
| Salicylate de zinc | 2 g |
| Dodecyl polyglucoside | 4 g |
| Lauryether sulfosuccinate 2Na | 9 g |
| Cocoamphodiacetate | 1,7 g |
| Alkylpolypeptide de Blé | 1,2 g |
| Polymère démêlant | QS |
| Elfacos GT 282 | QS |
| Cocamide MEA | QS |
| Parfum | QS |
| Eau déminéralisée QSP | 100 g |

**Revendications**

1. Utilisation de la néoruscine pour la fabrication d'une composition capillaire pharmaceutique, cosmétique ou dermocosmétique destinée à traiter l'alopécie.

2. Utilisation selon la revendication 1, **caractérisée en ce que** le néoruscine est préparée par extraction à partir de *Ruscus aculeatus* impliquant une opération de concentration suivi d'un ajout d'un alcool de C3 à C4, tel que le butanol-1 ou l'isopropanol.

3. Utilisation de la néoruscine pour la fabrication d'une composition capillaire dermatologique destinée à traiter l'alopécie, **caractérisée en ce que** l'administration journalière varie de 5 à 10 mg.

4. Méthode de traitement cosmétique pour lutter contre la chute des cheveux, **caractérisée en ce qu'**elle consiste à administrer par voie topique une composition capillaire contenant de la néoruscine pour une administration journalière variant de 1 à 3 mg.

# FIGURE 1

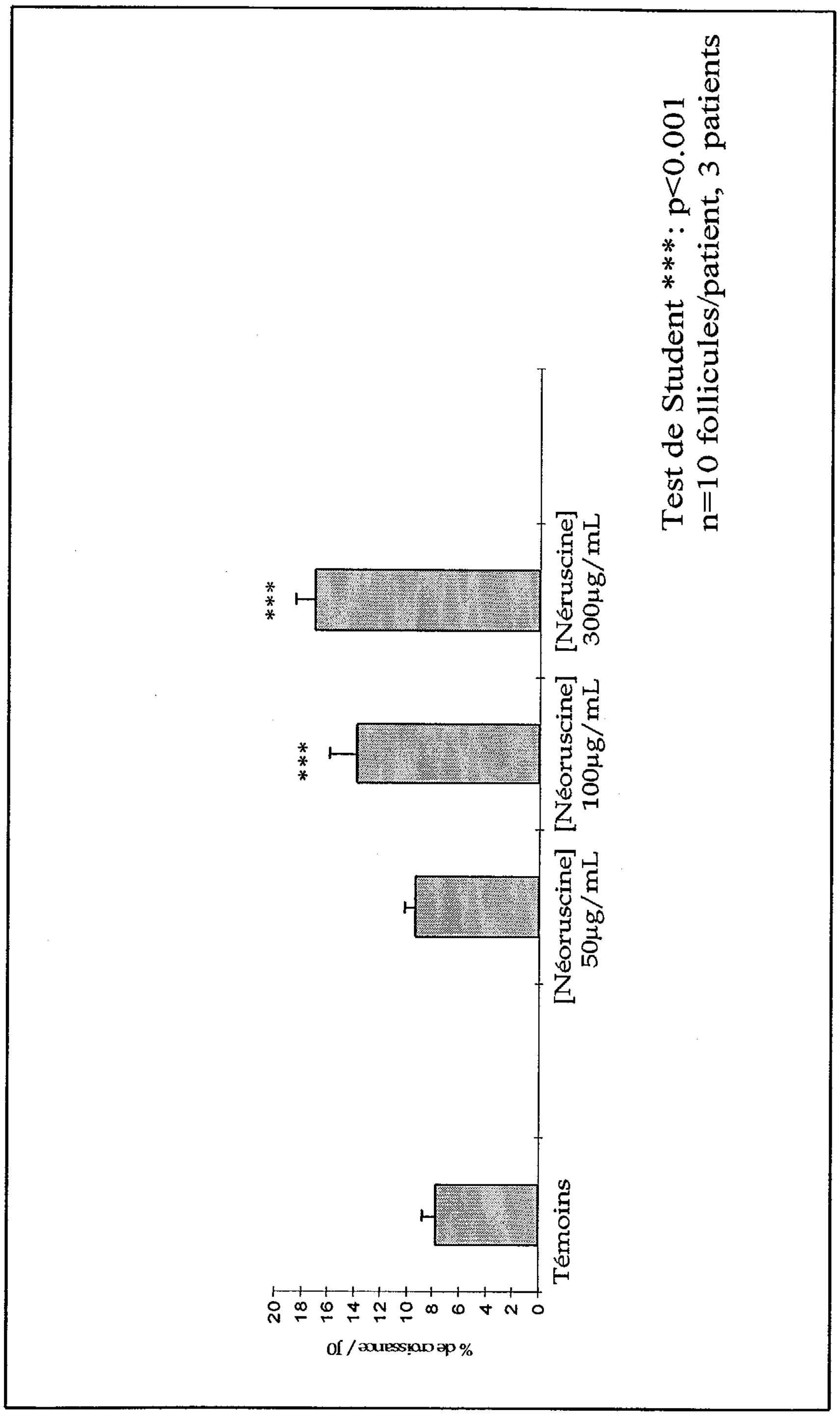
% de croissance / J0
0
2
4
6
8
10
12
14
16
18
20
Témoins
[Néoruscine] 50µg/mL
***
[Néoruscine] 100µg/mL
***
[Néruscine] 300µg/mL
Test de Student ***: p<0.001
n=10 follicules/patient, 3 patients

Office européen des brevets

**RAPPORT PARTIEL DE RECHERCHE EUROPEENNE**
qui selon la règle 45 de la Convention sur le brevet européen est consideré, aux fins de la procédure ultérieure, comme le rapport de la recherche européenne

Numéro de la demande
EP 03 29 0452

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X<br>Y | FR 2 779 348 A (FABRE PIERRE DERMO COSMETIQUE) 10 décembre 1999 (1999-12-10)<br>le document en entier, surtout pages 2-5, page 7 lignes 17-32, page 8 lignes 1-20, page 9 lignes 8-15, exemples et revendications 1-6,11,12<br>--- | 1,4<br>2 | A61K31/7048<br>A61K35/78<br>A61K7/06<br>A61P17/14 |
| Y | FR 2 258 175 A (FABRE SA PIERRE) 18 août 1975 (1975-08-18)<br>* le document en entier *<br>--- | 2 | |
| A | CA 2 075 592 A (SLAMKO DOUGLAS) 8 février 1994 (1994-02-08)<br>le document en entier, surtout page 2 ligne 12 et page 3 lignes 2,3,21<br>--- | 1-4 | |
| A | WO 01 72266 A (PIERRO FRANCESCO DI ;INDENA SPA (IT)) 4 octobre 2001 (2001-10-04)<br>le document en entier, surtout page 2 ligne 21, page 5 ligne 13, page 6 ligne 8 et page 7 ligne 8<br>---<br>-/-- | 1-4 | DOMAINES TECHNIQUES RECHERCHES (Int.Cl.7)<br>A61K |

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

Bien que la revendication 4 concerne une méthode de traitement du corps humain/animal (Article 52(4) CEB), la recherche a été effectuée et basée sur les effets imputés au produit/à la composition.

| Lieu de la recherche | Date d'achèvement de la recherche | Examinateur |
|---|---|---|
| LA HAYE | 10 juin 2003 | Gac, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

Office européen des brevets

**RAPPORT PARTIEL DE RECHERCHE EUROPEENNE**

Numero de la demande
EP 03 29 0452

| DOCUMENTS CONSIDERES COMME PERTINENTS | | | CLASSEMENT DE LA DEMANDE (Int.Cl.7) |
|---|---|---|---|
| Catégorie | Citation du document avec indication, en cas de besoin, des parties pertinentes | Revendication concernée | |
| A | "RUSCUS ACULEATUS (BUTCHER'S BROOM)" ALTERNATIVE MEDICINE REVIEW, THORNE RESEARCH INC., SANDPOINT,, US, vol. 6, no. 6, décembre 2001 (2001-12), pages 608-612, XP008009565 ISSN: 1089-5159 * le document en entier * --- | 1,2 | |
| A | POURRAT H ET AL: "ISOLEMENT ET CONFIRMATION DE LA STRUCTURE PAR RMN DU 13C DE LA PRINCIPLE PROSAPOGENINE DE RUSCUS ACULEATUS L" ANNALES PHARMACEUTIQUES FRANCAISES, MASSON, PARIS, FR, vol. 40, no. 5, 1982, pages 451-458, XP000961294 ISSN: 0003-4509 * le document en entier * ----- | 2 | DOMAINES TECHNIQUES RECHERCHES (Int.Cl.7) |

EPO FORM 1503 03.82 (P04C11)

## ANNEXE AU RAPPORT DE RECHERCHE EUROPEENNE RELATIF A LA DEMANDE DE BREVET EUROPEEN NO.

EP 03 29 0452

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
*Les renseignements fournis sont donnés à titre indicatif et n'engagent pas la responsabilité de l'Office européen des brevets.*

10-06-2003

| Document brevet cité au rapport de recherche | | Date de publication | Membre(s) de la famille de brevet(s) | | Date de publication |
|---|---|---|---|---|---|
| FR 2779348 | A | 10-12-1999 | FR 2779348 | A1 | 10-12-1999 |
| | | | AT 216588 | T | 15-05-2002 |
| | | | DE 69901343 | D1 | 29-05-2002 |
| | | | DE 69901343 | T2 | 02-01-2003 |
| | | | EP 1082125 | A1 | 14-03-2001 |
| | | | ES 2174610 | T3 | 01-11-2002 |
| | | | WO 9962529 | A1 | 09-12-1999 |
| | | | PT 1082125 | T | 30-09-2002 |
| FR 2258175 | A | 18-08-1975 | FR 2258175 | A1 | 18-08-1975 |
| | | | DE 2436479 | A1 | 24-07-1975 |
| CA 2075592 | A | 08-02-1994 | CA 2075592 | A1 | 08-02-1994 |
| WO 0172266 | A | 04-10-2001 | IT MI20000628 | A1 | 24-09-2001 |
| | | | AU 3737501 | A | 08-10-2001 |
| | | | CN 1419436 | T | 21-05-2003 |
| | | | CZ 20023161 | A3 | 12-02-2003 |
| | | | WO 0172266 | A1 | 04-10-2001 |
| | | | EP 1265586 | A1 | 18-12-2002 |
| | | | NO 20024519 | A | 25-11-2002 |
| | | | US 2002197290 | A1 | 26-12-2002 |
| | | | US 2001033849 | A1 | 25-10-2001 |

EPO FORM P0460

Pour tout renseignement concernant cette annexe : voir Journal Officiel de l'Office européen des brevets, No.12/82